# EUROPÄISCHE PATENTSCHRIFT

(11) **EP 1 307 460 B1**
(45) Veröffentlichungstag und Bekanntmachung des Hinweises auf die Patenterteilung: **03.03.2004**
(21) Anmeldenummer: 01956566.2
(22) Anmeldetag: 26.07.2001
(51) Int. Cl.: C07D 491/14, C07D 487/04, C07D 263/30, C07D 317/48, C07D 413/10, C07C 69/614

(54) **VERFAHREN ZUR HERSTELLUNG VON IMIDAZO [1,2-c] [2,3]BENZODIAZEPINEN UND ZWISCHENPRODUKTE BEI DEREN HERSTELLUNG**
METHOD FOR THE PRODUCTION OF IMIDAZO-[1,2-c][2,3]-BENZODIAZEPINES AND INTERMEDIATES IN THE PRODUCTION THEREOF
PROCEDE DE FABRICATION D'IMIDAZO[1,2-c][2,3]BENZODIAZEPINES ET PRODUITS INTERMEDIAIRES POUR CETTE FABRICATION

(30) Priorität: 10.08.2000 DE 10041671
(43) Veröffentlichungstag der Anmeldung: 07.05.2003
(73) Patentinhaber: Schering Aktiengesellschaft, 13353 Berlin (DE)
(72) Erfinder: WINTER, Eric, 38108 Braunschweig (DE); SCHNEIDER, Matthias, 10589 Berlin (DE); TILSTAM, Ulf, 1970 Wezembeek Oppem (BE)
(86) Internationale Anmeldenummer: PCT/EP2001/008661
(87) Internationale Veröffentlichungsnummer: WO 2002/012247

(56) Entgegenhaltungen:
- WO-A-97/28163
- WO-A-99/06408
- F GATTA ET AL: "Derivatives of 2,3-Benzodiazepine" IL FARMACO - ED. SC, Bd. 40, 1985, Seiten 942-955, XP002902060
- H H WASSERMAN ET AL: "Oxazoles as masked activated carboxylates. Synthesis of (+)-di-o-methyl-culvularin" TETRAHEDRON LETTERS, Bd. 22, Nr. 48, 1981, Seiten 4849-4852, XP002902061
- H H WASSERMAN ET AL: "Activated carboxylates from the photooxygenation of oxazoles" TETRAHEDRON, Bd. 37, Nr. 23, 1981, Seiten 4059-4067, XP002902062
- ANGELA DE SARRO ET AL: "Synthesis and anticonvulsant activity of new 2,3-benzobiazepines as AMPA receptor antagonists" IL FARMACO, Bd. 54, Nr. 3, 1999, Seiten 178-187, XP002902063
- YAN WANG ET AL: "Synthesis of 7,8-(methylenedioxy)-1-phenyl-3,5-dihydro- 4H-2,3-benzodiazepin-4-ones as novel and potent noncompetitive AMPA receptor antagonists" J. MED. CHEM., Bd. 41, Nr. 14, 1998, Seiten 2621-2625, XP002902064
- ANGELA DE SARRO ET AL: "7,8-methylenedioxy-4H-2,3-benzodiazepin-4 -ones as novel AMPA receptor antagonists" BIOORGANIC & MEDICINAL CHEMISTRY LETTERS, Bd. 8, Nr. 8, 1998, Seiten 971-976, XP002902065
- GIZELLA ABRAHAM ET AL: "New non competetive AMPA antagonists" BIOORGANIC & MEDICINAL CHEMISTRY, Bd. 8, Nr. 8, 2000, Seiten 2127-2143, XP002902066

## Beschreibung

Die Erfindung betrifft ein neues Verfahren zur Herstellung von Imidazo-[1,2-c][2,3]benzodiazepinen der allgemeinen Formel (**1**) sowie neuer Zwischenprodukte im Verfahren, 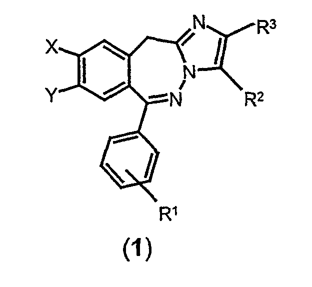 worin
R¹ = Wasserstoff, C₁-C₆-Alkyl, Nitro, Halogen, Cyano, C₁-C₄-Alkoxy, -CF₃, Hydroxy oder C₁-C₆-Alkanoyloxy,
R² und R³ gleich oder verschieden sind und Wasserstoff, Halogen, C₁-C₆-Alkoxy, Hydroxy, Cyano, C₁-C₆-Alkanoyl, C₂-C₆-Alkynyl, C₂-C₆-Alkenyl, gegebenenfalls durch Halogen, Hydroxy oder C₁₋₆-Alkoxy substituiertes C₁-C₆-Alkyl, C₃-C₇-Cycloalkyl oder einen gegebenenfalls mit Halogen, C₁₋₄-Alkoxy oder C₁₋₄-Alkyl substituierten Aryl- oder Hetarylrest,
X = Wasserstoff oder Halogen,
Y = C₁-C₆-Alkoxy oder
X und Y gemeinsam -O-(CH₂)ₙ-O- mit
n = 1, 2 oder 3 bedeuten.

Die Erfindung beinhaltet auch als neue Zwischenprodukte zur Herstellung pharmakologisch wirksamer Verbindungen Phenylessigsäurederivate der allgemeinen Formel **5**, 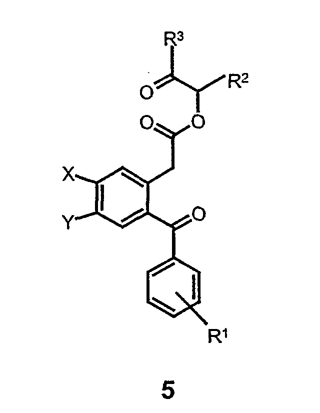 worin
R¹ = Wasserstoff, C₁-C₆-Alkyl, Nitro, Halogen, Cyano, C₁-C₄-Alkoxy, -CF₃, Hydroxy oder C₁-C₆-Alkanoyloxy,
R² und R³ gleich oder verschieden sind und Wasserstoff, Halogen, C₁-C₆-Alkoxy, Hydroxy, Cyano, C₁-C₆-Alkanoyl, C₂-C₆-Alkynyl, C₂-C₆-Alkenyl, gegebenenfalls durch Halogen, Hydroxy oder C₁₋₆-Alkoxy substituiertes C₁-C₆-Alkyl, C₃-C₇-Cycloalkyl oder einen gegebenenfalls mit Halogen, C₁₋₄-Alkoxy oder C₁₋₄-Alkyl substituierten Aryl- oder Hetarylrest,
X = Wasserstoff oder Halogen,
Y = C₁-C₆-Alkoxy oder
X und Y gemeinsam -O-(CH₂)ₙ-O- mit
n = 1, 2 oder 3 bedeuten
und
Oxazol-Derivate der allgemeinen Formel **6**, 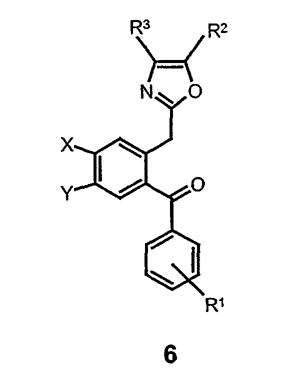 worin
R¹ = Wasserstoff, C₁-C₆-Alkyl, Nitro, Halogen, Cyano, C₁-C₄-Alkoxy, -CF₃, Hydroxy oder C₁-C₆-Alkanoyloxy,
R² und R³ gleich oder verschieden sind und Wasserstoff, Halogen, C₁-C₆-Alkoxy, Hydroxy, Cyano, C₁-C₆-Alkanoyl, C₂-C₆-Alkynyl, C₂-C₆-Alkenyl, gegebenenfalls durch Halogen, Hydroxy oder C₁₋₆-Alkoxy substituiertes C₁-C₆-Alkyl, C₃-C₇-Cycloalkyl oder einen gegebenenfalls mit Halogen, C₁₋₄-Alkoxy oder C₁₋₄-Alkyl substituierten Aryl- oder Hetarylrest,
X = Wasserstoff oder Halogen,
Y = C₁-C₆-Alkoxy oder
X und Y gemeinsam -O-(CH₂)ₙ-O- mit
n = 1, 2 oder 3 bedeuten.

Die Reste innerhalb der allgemeinen Formeln haben folgende Bedeutungen:

Unter C₁-C₆-Alkyl ist jeweils ein geradkettiger oder verzweigter Alkylrest wie beispielsweise Methyl, Ethyl, Propyl, Isopropyl, Butyl, Isobutyl, sek. Butyl, Pentyl, Isopentyl oder Hexyl zu verstehen.

R² und R³ in der Bedeutung C₂-C₆-Alkenyl enthalten jeweils mindestens eine Doppelbindung, wie beispielsweise Vinyl, Propenyl, Buten-1-yl, Isobutenyl, Penten-1-yl, 2,2-Dimethyl-buten-1-yl, 3-Methylbuten-1-yl, Hexen-1-yl.

Bedeuten R² und R³ C₁-C₆-Alkynyl, so ist mindestens eine Dreifachbindung vorhanden, wie beispielsweise Ethynyl, Propynyl, Butyn-1-yl, Butyn-2-yl, Pentyl-1-yl, Pentyl-2-yl, 3-Methylbutyn-1-yl, Hexyn-1-yl. Die vorstehend beschriebenen Alkenyl- oder Alkinylreste können gegebenenfalls auch mit Halogenatomen substituiert sein. Liegt ein halogenierter Alkylrest vor, so kann dieser ein- oder mehrfach halogeniert, aber auch perhalogeniert sein, wie beispielsweise -CF₃.

Unter Halogen innerhalb der vorstehenden Resten ist jeweils Fluor, Chlor, Brom und Jod zu verstehen.

R² und R³ in der Bedeutung Aryl- und Hetarylrest können gegebenenfalls ein-, zwei- oder dreifach mit Halogen, C₁₋₄-Alkoxy-, oder C₁₋₄-Alkylresten substituiert sein; beliebige Permutationen sind möglich.

Der Aryl- und Hetarylrest kann als Mono- oder Bicyclus vorliegen und 5 - 12 Ringatome, vorzugsweise 5 - 9 Ringatome enthalten, wie beispielsweise Phenyl, Biphenyl, Naphthyl, Indenyl als Arylrest und Thienyl, Furyl, Pyranyl, Pyrrolyl, Pyrazolyl, Pyridyl, Pyrimidyl, Pyridazinyl, Oxazolyl, Iso-oxazolyl, Thiazolyl, Isothioazolyl, 1,3,4-Oxadiazol-2-yl, 1,2,4-Oxadiazol-5-yl, 1,2,4-Oxadiazol-3-yl, Chinolyl, Isochinolyl, Benzo[1]thienyl, Benzofuranyl als Hetarylrest, enthaltend 1-3 Heteroatomen wie beispielsweise Schwefel, Sauerstoff, und/oder Stickstoff. Als bevorzugte Reste sind 2-Thienyl, 3-Thienyl, Pyridin-2-yl, Pyridin-3-yl, Pyridin-4-yl und Phenyl genannt.

Mit R² und R³ in der Bedeutung C₃-C₈-Cycloalkyl sind beispielsweise der Cyclopropyl-, Cyclobutyl-, Cyclopentyl-, Cyclohexyl- und Cycloheptylrest gemeint.

Als C₁-C₆-Alkanoylreste sind jeweils geradkettige oder verzweigte aliphatische Carbonsäurereste wie beispielsweise Formyl, Acetyl, Propinoyl, Butanoyl, Isopropylcarbonyl, Caproyl, Valeroyl oder Trimethylacetyl geeignet.

Bevorzugt bedeuten R¹ Wasserstoff, Chlor, Nitro, Methoxy; R² und R³ Wasserstoff oder C₁₋₄-Alkyl oder Phenyl; X und Y gemeinsam -O-CH₂-O-.

Verfahren zur Herstellung der Verbindungen der allgemeinen Formel 1 werden innerhalb der WO 97/28163 beschrieben.

Aufgabe dieser Erfindung ist ein neues Verfahren zur Synthese der Verbindungen der allgemeinen Formel **1.** Ebenfalls Gegenstand dieser Erfindung sind neue, bisher nicht bekannte Zwischenprodukte der allgemeinen Formeln **5** und **6**, die im Rahmen der Synthese durchlaufen werden und per se oder derivatisiert als Ausgangsmaterialien für die Synthese anderer Zielmoleküle verwendet werden können.

Gelöst wird diese Aufgabe durch die Lehre der Patentansprüche.

Durch das erfindungsgemäße Verfahren werden weniger Zwischenstufen durchlaufen als in der bekannten Synthese aus dem Stand der Technik, die Anzahl der Reinigungsschritte ist deutlich niedriger und die Gesamtausbeute wird erhöht. Das erfindungsgemäße Verfahren ermöglicht die Herstellung der Verbindungen der Formel I in technischem Maßstab.

Die Erfindung beinhaltet also ein Verfahren zur Herstellung von Imidazo[1,2-c][2,3]benzodiazepinen der allgemeinen Formel **1**
worin R¹, R² und R³, sowie X und Y die vorstehende Bedeutung haben,
aus Phenylessigsäuren der allgemeinen Formel **4,** 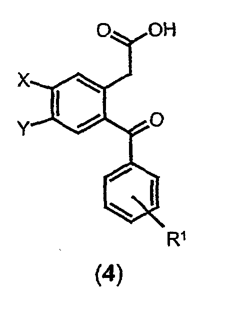 worin X, Y und R¹ die oben genannte Bedeutung haben,
a) durch Veresterung mit einem Alkohol der Formel **5 a** 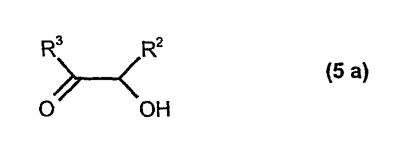 zum Phenylessigester der allgemeinen Formel **5**, 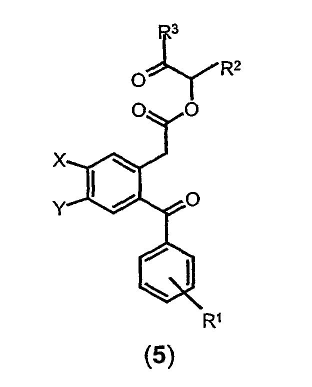 worin
   X, Y, R¹, R² und R³ die oben genannte Bedeutung haben,
b) durch Kondensation mit Ammoniak oder einem Ammoniak-Donor zu dem Oxazol der allgemeinen Formel **6**, 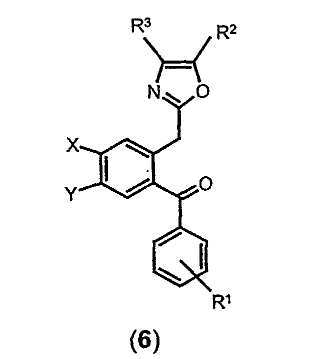
worin
X, Y, R¹, R² und R³ die oben genannte Bedeutung haben und anschließender

### Hydrazinolyse zu den Verbindungen der allgemeinen Formel 1.

Imidazo[1,2-c][2,3]benzodiazepine der allgemeinen Formel **1** werden gemäß Schema 1 synthetisiert.

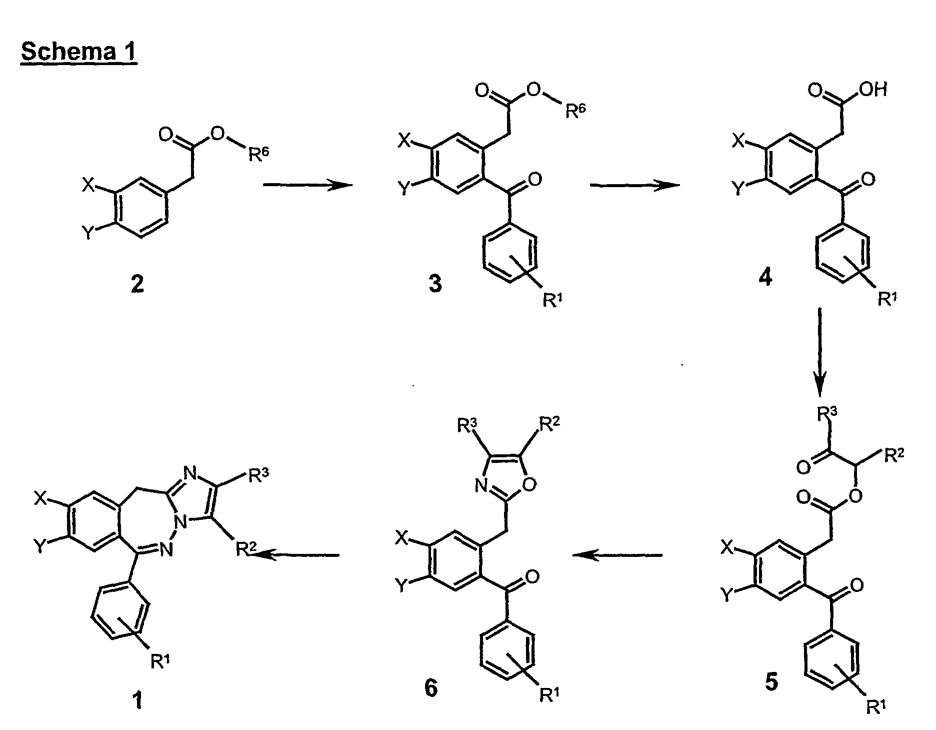

Die Umsetzung einer Verbindung der allgemeinen Formel **2** zu einer Verbindung der allgemeinen Formel **3** erfolgt nach einem an sich bekannten Verfahren (z. B. *J. Chem. Soc., Perkin Trans. 1* **1991**, 169 - 173) einer Friedel-Crafts-Reaktion. Beispielsweise werden Verbindungen der allgemeinen Formel **2** in Gegenwart von Lewissäuren wie etwa Zinntetrachtorid, Aluminiumtrichlorid, Titantetrachlorid und einem Acylierungsmittel wie etwa Benzoylchlorid, Benzoesäureanhydrid oder einem anderen aktivierten Carbonsäurederivat umgesetzt. Eine wesentliche Ausbeuteerhöhung wird erzielt, wenn zusätzlich *N*,*N*-Dimethylacetamid zum Reaktionsgemisch hinzugefügt wird. Als Lösemittel können halogenierte Kohlenwasserstoffe wie z. B. Methylenchlorid oder Ethylenchlorid und deren Gemische dienen. Die Reaktion wird in einem Temperaturbereich von -30 ° bis 50 °C durchgeführt, bevorzugt jedoch im Temperaturbereich von 0 ° bis 25 °C.

Die Umsetzung einer Verbindung der allgemeinen Formel **3** zu einer Verbindung der allgemeinen Formel **4** erfolgt nach einem an sich bekannten Verfahren einer Verseifungsreaktion. Beispielsweise wird eine Verbindung der allgemeinen Formel **3** in Gegenwart einer Base wie Alkalihydroxid, bevorzugt aber Natriumhydroxid, in einem Lösemittel wie einem niederen, bevorzugt primären Alkohol oder Wasser oder deren Gemische erhitzt. Die Reaktion wird in einem Temperaturbereich von 25 ° bis 150 °C durchgeführt, bevorzugt jedoch im Temperaturbereich von 70 ° bis 110 °C.

Die Umsetzung einer Verbindung der allgemeinen Formel **4** zu einer Verbindung der Formel **5** erfolgt nach einem an sich bekannten Verfahren einer Veresterung. Beispielsweise wird eine Verbindung der allgemeinen Formel **4** in Gegenwart eines Aktivierungsreagenzes wie z. B. Carbonyldiimidazol und einem Alkohol der Formel 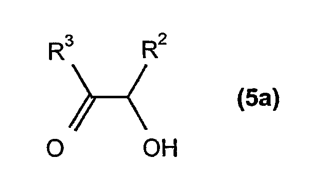 wie 3-Hydroxy-2-butanon umgesetzt. Als Lösemittel können halogenierte Kohlenwasserstoffe wie z. B. Methylenchlorid oder Ethylenchlorid sowie THF und deren Gemische dienen. Die Reaktion wird in einem Temperaturbereich von -20 ° bis 100 °C durchgeführt, bevorzugt jedoch im Temperaturbereich von 0 ° bis 25 °C.

Dem Fachmann ist geläufig, dass R² und R³ in Verbindungen der allgemeinen Formel **5** nach Standardmethoden variiert werden können. Dies kann durch Verwendung anderer Alkohole im Veresterungsschritt geschehen, aber auch durch Umesterung eines schon vorhandenen Esters. R² und R³ kann also die Bedeutung von Wasserstoff, Halogen, Alkoxy, Hydroxy, Cyano, Alkanoyl, gegebenenfalls substituiertes Alkynyl, gegebenenfalls substituiertes Alkenyl, gegebenenfalls durch Halogen, Hydroxy, Alkoxy, substituiertes Alkyl, Cycloalkyl oder einen gegebenenfalls substituierten Aryl- oder Hetarylrest besitzen.

Die Umsetzung einer Verbindung der allgemeinen Formel 5 zu einer Verbindung der allgemeinen Formel **6** erfolgt nach einem an sich bekannten Verfahren zur Herstellung von Oxazolen durch Kondensation zweier Carbonyl-Gruppen mit Ammoniak. Beispielsweise wird eine Verbindung der allgemeinen Formel **5** in Gegenwart von Ammoniumacetat, Ammoniak, einer Ammoniak-Lösung oder einem anderen Ammoniak-Donor wie z. B. Acetamid oder Formamid in Gegenwart von Essigsäure umgesetzt. Als Lösemittel können halogenierte Kohlenwasserstoffe wie z. B. Methylenchlorid oder Ethylenchlorid, organische Säuren wie z. B. Ameisensäure oder Essigsäure sowie niederen Alkoholen wie z. B. Methanol oder Ethanol, aber auch Ethylenglykol und deren Gemische dienen. Die Reaktion wird in einem Temperaturbereich von 0 ° bis 150 °C durchgeführt, bevorzugt jedoch im Temperaturbereich von 50 ° bis 100 °C.

Die Umsetzung einer Verbindung der allgemeinen Formel **6** zu einer Verbindung der allgemeinen Formel **1** erfolgt nach einem an sich bekannten Verfahren durch Hydrazinolyse bzw. Hydrazonbildung. Beispielsweise wird eine Verbindung der allgemeinen Formel **6** in Gegenwart von Hydrazin oder Hydrazin-Hydrat umgesetzt. Als Lösemittel können halogenierte Kohlenwasserstoffe wie z. B. Methylenchlorid oder Ethylenchlorid, organische Säuren wie z. B. Ameisensäure oder Essigsäure sowie niederen Alkoholen wie z. B. Methanol oder Ethanol, aber auch Ethylenglykol und deren Gemische dienen. Die Reaktion wird in einem Temperaturbereich von 0 ° bis 200 °C durchgeführt, bevorzugt jedoch im Temperaturbereich von 80 ° bis 120 °C. Eine Reaktionsführung in einem Autoklaven ist möglich.

Die Umsetzung einer Verbindung der allgemeinen Formel **5** zu einer Verbindung der Formel **1** erfolgt auch in einer Eintopfreaktion. Beispielsweise wird eine Verbindung der Formel **5** in Gegenwart von Ammoniumacetat, Ammoniak, einer Ammoniak-Lösung oder einem anderen Ammoniak-Donor wie z. B. Acetamid oder Formamid in Gegenwart von Essigsäure umgesetzt. Als Lösemittel können halogenierte Kohlenwasserstoffe wie z. B. Methylenchlorid oder Ethylenchlorid, organische Säuren wie z. B. Ameisensäure oder Essigsäure sowie niederen Alkoholen wie z. B. Methanol oder Ethanol, aber auch Ethylenglykol und deren Gemische dienen. Die Reaktion wird in einem Temperaturbereich von 0 ° bis 150 °C durchgeführt, bevorzugt jedoch im Temperaturbereich von 50 ° bis 100°C. Nach erfolgter Reaktion werden zum Reaktionsgemisch Hydrazin oder Hydrazin-Hydrat zugesetzt. Die Reaktion wird in einem Temperaturbereich von 0° bis 200 °C durchgeführt, bevorzugt jedoch im Temperaturbereich von 80 ° bis 120 °C. Eine Reaktionsführung in einem Autoklaven ist möglich.

Soweit die Herstellung der Ausgangsverbindungen nicht beschrieben wird, sind diese bekannt oder analog zu bekannten oder hier beschriebenen Verfahren herstellbar. Nachfolgend wird das erfindungsgemäße Verfahren beispielsmäßig dargestellt.

### Beispiele:

### 2-Benzoyl-4,5-(methylendioxy)-phenylessigsäuremethylester (3)

79 ml Zinntetrachlorid und 25 ml N,N'-Dimethylacetamid in 270 ml Methylenchlorid werden bei Raumtemp. mit 39 ml Benzoylchlorid versetzt. Zu diesem Gemisch werden bei 0°C 44 g 4,5-(Methylendioxy)-phenylessigsäuremethylester (**2**) getropft und die Lösung wird 12 h bei Raumtemp. gerührt. Zwecks Aufarbeitung folgt die Zugabe von 660 ml Wasser bei -15°C sowie eine Extraktion der wäßrigen Phase mit Methylenchlorid. Die gesammelten organischen Fraktionen werden mit 6 N wäßr. NaOH gewaschen und vollständig am Rotationsverdampfer eingeengt. Das Rohprodukt **3** (74 g) wird aus Ethanol kristallisiert.

¹H-NMR (CDCl₃): δ = 3.61 (s, 3 H, OMe), 3.80 (s, 2 H, benzyl. CH₂), 6.03 (s, 2 H, OCH₂O), 6.84 und 6.88 (2 s à 1 H, 3- und 6-H Aryl), 7.43-7.80 (m, 5 H, PhCO).

| | | | |
|---|---|---|---|
| Smp.: 74-76°C, Verbrennungsanalyse | Ber. | C 68.45 | H 4.73 |
| | Gef. | C 68.53 | H 4.59 |

In analoger Weise werden hergestellt:
2-(4-Chlor-benzoyl)-4,5-(methylendioxy)-phenylessigsäuremethylester
2-(4-Nitro-benzoyl)-4,5-(methylendioxy)-phenylessigsäuremethylester
2-(4-Methoxy-benzoyl)-4,5-(methylendioxy)-phenylessigsäuremethylester
2-(4-Methyl-benzoyl)-4,5-(methylendioxy)-phenylessigsäuremethylester
2-(4-Cyano-benzoyl)-4,5-(methylendioxy)-phenylessigsäuremethylester

### 2-Benzoyl-4,5-(methylendioxy)-phenylessigsäure (4)

Eine Suspension von 10 g **2** in 50 ml 1 N wäßr. NaOH wird 2 h unter Rückfluß erhitzt. Dann werden 10 ml 1 N wäßr. Schwefelsäure zugegeben, der ausgefallende Feststoff wird abfiltriert und mit Wasser nachgewaschen. Das Produkt **4** (8.3 g) wird i. V. getrocknet und ohne weitere Reinigung in die nächste Stufe eingesetzt.

¹H-NMR (DMSO): δ = 3.68 (s, 2 H, benzyl. CH₂), 6.12 (s, 2 H, OCH₂O), 6.86 und 7.03 (2 s ä 1 H, 3- und 6-H Aryl), 7.50-7.73 (m, 5 H, PhCO), 12.18 (br. s, 1 H, COOH).

| | | | |
|---|---|---|---|
| Smp.: 185-189°C, Verbrennungsanalyse | Ber. | C 67.60 | H 4.25 |
| | Gef. | C 67.66 | H 4.20 |

In analoger Weise werden hergestellt:
2-(4-Chlor-benzoyl)-4,5-(methylendioxy)-phenylessigsäure
2-(4-Nitro-benzoyl)-4,5-(methylendioxy)-phenylessigsäure
2-(4-Methoxy-benzoyl)-4,5-(methylendioxy)-phenylessigsäure
2-(4-Methyl-benzoyl)-4,5-(methylendioxy)-phenylessigsäure
2-(4-Cyano-benzoyl)-4,5-(methylendioxy)-phenylessigsäure

### 2-Benzoyl-4,5-(methylendioxy)-phenylessigsäure-(3-oxo-but-2-ylester) (5)

50 g **4,** 19 g 3-Hydroxy-2-butanon und 32 g N,N'-Carbonyldiimidazol werden bei Raumtemp. in 500 ml Methylenchlorid gelöst und 3 d gerührt. Zur Aufarbeitung wird mit 200 ml VE-Wasser versetzt und anschließend mit Methylenchlorid extrahiert. Die gesammelten organischen Fraktionen werden am Rotationsverdampfer eingeengt. Das Rohprodukt wird aus Methanol (2fach) kristallisiert, es werden 45 g **5** erhalten.

¹H-NMR (CDCl₃): δ = 1.32 (d, 3 H, *J* = 6.9, CH₃C-O), 2.11 (s, 3 H, CH₃C=O), 3.88 (s, 2 H, benzyl. CH₂), 5.03 (q, 1 H, *J* = 6.9, CH-O), 6.04 (s, 2 H, OCH₂O), 6.87 und 6.90 (2 s à 1 H, 3- und 6-H Aryl), 7.42-7.74 (m, 5 H, PhCO).

| | | | |
|---|---|---|---|
| Smp.: 81-83°C, Verbrennungsanalyse | Ber. | C 67.79 | H 5.12 |
| | Gef. | C 67.83 | H 5.04 |

In analoger Weise werden hergestellt:
2-(4-Chlor-benzoyl)-4,5-(methylendioxy)-phenylessigsäure-(3-oxo-but-2-ylester)
2-(4-Nitro-benzoyl)-4,5-(methylendioxy)-phenylessigsäure-(3-oxo-but-2-ylester)
2-(4-Methoxy-benzoyl)-4,5-(methylendioxy)-phenylessigsäure-(3-oxo-but-2-ylester)
2-Benzoyl-4,5-(methylendioxy)-phenylessigsäure-(4-oxo-hex-3-ylester)
2-Benzoyl-4,5-(methylendioxy)-phenylessigsäure-(2-oxo-1,2-diphenyl-eth-1-ylester)

### 2-Benzoyl-4,5-(methylendioxy)-benzyl-4,5-dimethyl-oxazol (6)

Zu 10 g **5** und 19 g Ammoniumacetat in 200 ml 1 ,2-Dichlorethan werden 14 ml konz. Essigsäure gegeben und das Gemisch wird 6 h unter Rückfluß erhitzt. Dann werden 100 ml einer 1 N wäßr. NaOH hinzugefügt und es wird mit Methylenchlorid extrahiert. Die vereinigten organischen Extrakte werden am Rotationsverdampfer eingedampft und das Rohprodukt über Kieselgel filtriert (Hexan:Essigester 9:1 → 8:2). Es werden 6.9 g reines **6** erhalten (viskoses Öl).

¹H-NMR (CDCl₃): δ = 1.94 und 2.03 (2 s à 3 H, 2 CH₃), 4.13 (s, 2 H, benzyl. CH₂), 6.01 (s, 2 H, OCH₂O), 6.83 und 6.86 (2 s à 1 H, 3- und 6-H Aryl), 7.41-7.80 (m, 5 H, PhCO).

| | | | | |
|---|---|---|---|---|
| Verbrennungsanalyse | Ber. | C 71.63 | H 5.11 | N 4.18 |
| | Gef. | C 71.47 | H 5.04 | N 3.97 |

In analoger Weise werden hergestellt:
2-(4-Chlor-benzoyl)-4,5-(methylendioxy)-benzyl-4,5-dimethyl-oxazol
2-(4-Nitro-benzoyl)-4,5-(methylendioxy)-benzyl-4,5-dimethyl-oxazol
2-(4-Methoxy-benzoyl)-4,5-(methylendioxy)-benzyl-4,5-dimethyl-oxazol
2-Benzoyl-4,5-(methylendioxy)-benzyl-4,5-diethyl-oxazol
2-Benzoyl-4,5-(methylendioxy)-benzyl-4,5-diphenyl-oxazol

### 2,3-Dimethyl-6-phenyl-(12H)-[1,3]dioxolo[4,5-h]imidazo[1,2-c][2,3]benzodiazepin (1)

In einem Autoklaven werden zu 2 g **6**, gelöst in 18 ml Ethylenglykol und 2 ml konz. Essigsäure, 0.5 ml Hydrazin-Hydrat gegeben. Es wird 12 h auf 120°C erhitzt und anschließend das Reaktionsgemisch bei Raumtemp. mit 2 N wäßr. NaOH versetzt. Es wird mehrfach mit Essigester extrahiert und die vereinten organischen Phasen vollständig am Rotationsverdampfer eingeengt. Das Rohprodukt wird säulenchromatographisch gereinigt (Hexan:Essigester 9:1 → 8:2), die Ausbeute an reinem **1** beträgt 1.2 g.

¹H-NMR (DMSO): δ = 2.01 und 2.20 (2 s à 3 H, 2 CH₃), 3.82 (br. s, 2 H, benzyl. CH₂), 6.08 (s, 2 H, OCH₂O), 6.55 und 7.15 (2 s à 1 H, 2 Aryl-H), 7.48-7.72 (m, 5 H, PhCO).

| | | | | |
|---|---|---|---|---|
| Smp.: 177°C, Verbrennungsanalyse | Ber. | C 72.49 | H 5.18 | N 12.88 |
| | Gef. | C 72.33 | H 5.31 | N 12.46 |

Analog werden hergestellt:
2,3-Dimethyl-6-(4-chlor-phenyl)-(12*H*)-[1,3]dioxolo[4,5-h]imidazo[1,2-c][2,3] benzodiazepin
2,3-Dimethyl-6-(4-nitro-phenyl)-(12*H*)-[1,3]dioxolo[4,5-h]imidazo[1,2-c][2,3] benzodiazepin
2,3-Dimethyl-6-(4-methoxy-phenyl)-(12*H*)-[1,3]dioxolo[4,5-h]imidazo[1,2-c][2,3] benzodiazepin
2,3-Diethyl-6-phenyl-(12*H*)-[1,3]dioxolo[4,5-h]imidazo[1,2-c][2,3]benzodiazepin
2,3-Diphenyl-6-phenyl-(12*H*)-[1,3]dioxolo[4,5-h]imidazo[1,2-c][2,3]benzodiazepin

## Patentansprüche

1. Verfahren zur Herstellung von Imidazo[1,2-c][2,3]benzodiazepinen der allgemeinen Formel **1** 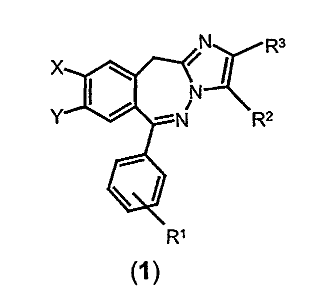 worin
R¹ = Wasserstoff, C₁-C₆-Alkyl, Nitro, Halogen, Cyano, C₁-C₄-Alkoxy, -CF₃, Hydroxy oder C₁-C₆-Alkanoyloxy,
R² und R³ gleich oder verschieden sind und Wasserstoff, Halogen, C₁-C₆-Alkoxy, Hydroxy, Cyano, C₁-C₆-Alkanoyl, C₂-C₆-Alkynyl, C₂-C₆-Alkenyl, gegebenenfalls durch Halogen, Hydroxy oder C₁₋₆-Alkoxy substituiertes C₁-C₆-Alkyl, C₃-C₇-Cycloalkyl oder einen gegebenenfalls mit Halogen, C₁₋₄-Alkoxy oder C₁₋₄-Alkyl substituierten Aryl- oder Hetarylrest,
X = Wasserstoff oder Halogen,
Y = C₁-C₆-Alkoxy oder
X und Y gemeinsam -O-(CH₂)ₙ-O- mit
n = 1, 2 oder 3 bedeuten,
aus Phenylessigsäuren der allgemeinen Formel **4**, 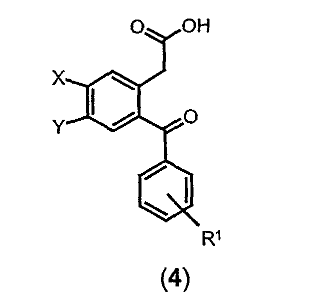 worin
X, Y und R¹ die oben genannte Bedeutung haben,
a) durch Veresterung mit einem Alkohol der Formel **5a** 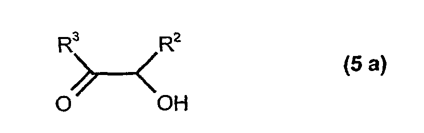 zum Phenylessigester der allgemeinen Formel **5,** 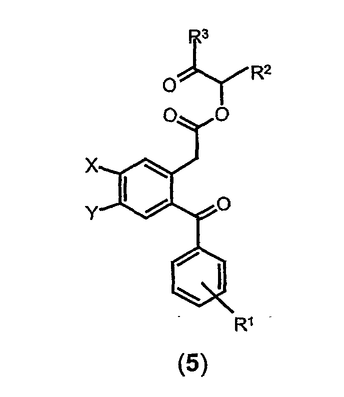 worin
X, Y, R¹, R² und R³ die oben genannte Bedeutung haben,
b) durch Kondensation mit Ammoniak oder einem Ammoniak-Donor zu dem Oxazol der allgemeinen Formel **6,** 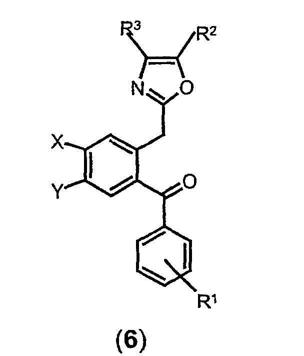 worin
X, Y, R¹, R² und R³ die oben genannte Bedeutung haben und anschließender Hydrazinolyse zu den Verbindungen der allgemeinen Formel **1** 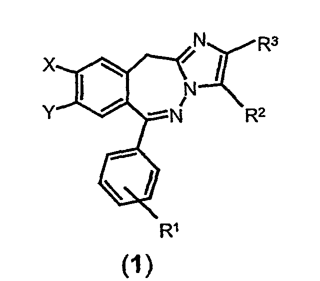 worin
X, Y, R¹, R² und R³ die oben genannte Bedeutung haben.

2. Verfahren nach Anspruch 1, wobei Verbindungen der allgemeinen Formel **5** mittels Eintopfreaktion durch Umsetzung in Gegenwart von Ammoniumacetat und Ammoniak oder einem Ammoniak-Donor bei 0 - 150 °C im Lösungsmittel oder Lösungsmittelgemischen und anschließend mit Hydrazin bei 0 - 200 °C, gegebenenfalls im Autoklaven, in die Verbindungen der allgemeinen Formel 1 umgesetzt werden.

3. Verfahren nach Anspruch 1 oder 2 zur Herstellung von 2,3-Dimethyl-6-phenyl-(12*H*)-[1,3]dioxolo[4,5-h]imidazo[1,2-c][2,3]benzodiazepin
2,3-Dimethyl-6-(4-chlor-phenyl)-(12*H*)-[1,3]dioxolo[4,5-h]imidazo[1,2-c][2,3] benzodiazepin
2,3-Dimethyl-6-(4-nitro-phenyl)- (12*H*)-[1,3]dioxolo[4,5-h]imidazo[1,2-c][2,3] benzodiazepin
2,3-Dimethyl-6-(4-methoxy-phenyl)- (12*H*)-[1,3]dioxolo[4,5-h]imidazo[1,2-c][2,3] benzodiazepin
2,3-Diethyl-6-phenyl-(12*H*)-[1,3]dioxolo[4,5-h]imidazo[1,2-c][2,3]benzodiazepin
2,3-Diphenyl-6-phenyl-(12*H*)-[1,3]dioxolo[4,5-h]imidazo[1,2-c][2,3]benzodiazepin

4. Phenylessigsäureester der allgemeinen Formel **5,** 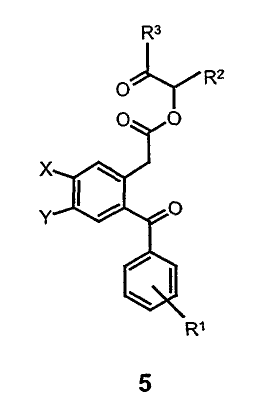 worin
R¹ = Wasserstoff, C₁-C₆-Alkyl, Nitro, Halogen, Cyano, C₁-C₄-Alkoxy, -CF₃, Hydroxy oder C₁-C₆-Alkanoyloxy,
R² und R³ gleich oder verschieden sind und Wasserstoff, Halogen, C₁-C₆-Alkoxy, Hydroxy, Cyano, C₁-C₆-Alkanoyl, C₂-C₆-Alkynyl, C₂-C₆-Alkenyl, gegebenenfalls durch Halogen, Hydroxy oder C₁₋₆-Alkoxy substituiertes C₁-C₆-Alkyl, C₃-C₇-Cycloalkyl oder einen gegebenenfalls mit Halogen, C₁₋₄-Alkoxy oder C₁₋₄-Alkyl substituierten Aryl- oder Hetarylrest,
X = Wasserstoff oder Halogen,
Y = C₁-C₆-Alkoxy oder
X und Y gemeinsam -O-(CH₂)ₙ-O- mit
n = 1, 2 oder 3 bedeuten.

5. Oxazol-Derivate der allgemeinen Formel **6,** 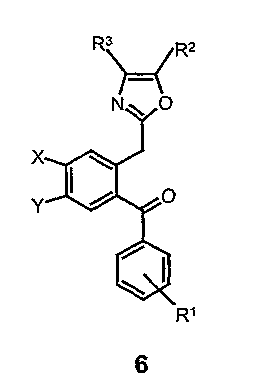 worin
R¹ = Wasserstoff, C₁-C₆-Alkyl, Nitro, Halogen, Cyano, C₁-C₄-Alkoxy, -CF₃, Hydroxy oder C₁-C₆-Alkanoyloxy,
R² und R³ gleich oder verschieden sind und Wasserstoff, Halogen, C₁-C₆-Alkoxy, Hydroxy, Cyano, C₁-C₆-Alkanoyl, C₂-C₆-Alkynyl, C₂-C₆-Alkenyl, gegebenenfalls durch Halogen, Hydroxy oder C₁₋₆-Alkoxy substituiertes C₁-C₆-Alkyl, C₃-C₇-Cycloalkyl oder einen gegebenenfalls mit Halogen, C₁₋₄-Alkoxy oder C₁₋₄-Alkyl substituierten Aryl- oder Hetarylrest,
X = Wasserstoff oder Halogen,
Y = C₁-C₆-Alkoxy oder
X und Y gemeinsam -O-(CH₂)ₙ-O- mit
n = 1, 2 oder 3 bedeuten.

## Claims

1. Process for the production of imidazo[1,2-c]-[2,3]benzodiazepines of general formula 1 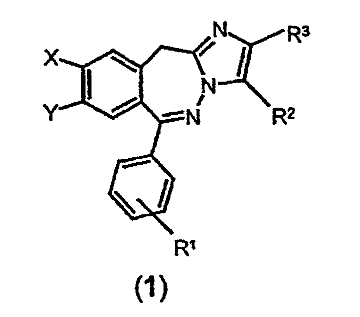 in which
R¹ = hydrogen, C₁-C₆-alkyl, nitro, halogen, cyano, C₁-C₄-alkoxy, -CF₃, hydroxy or C₁-C₆-alkanoyloxy,
R² and R³ are the same or different and mean hydrogen, halogen, C₁-C₆-alkoxy, hydroxy, cyano, C₁-C₆-alkanoyl, C₂-C₆-alkynyl, C₂-C₆-alkenyl; optionally halogen-, hydroxyl- or C₁₋₆-alkoxy-substituted C₁-C₆-alkyl, C₃-C₇-cycloalkyl; or an aryl or hetaryl radical that is optionally substituted by halogen, C₁₋₄-alkoxy or C₁₋₄-alkyl,
X = hydrogen or halogen,
Y = C₁-C₆-alkoxy or
X and Y together mean -O-(CH₂)ₙ-O- with
n = 1, 2 or 3,
from phenylacetic acids of general formula **4**, 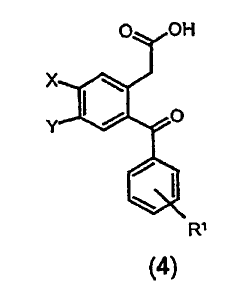 in which
X, Y and R¹ have the abovementioned meaning,
a) by esterification with an alcohol of formula **5a** 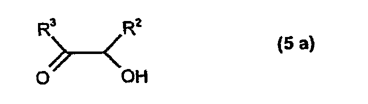 to form the phenylacetic ester of general formula **5**, 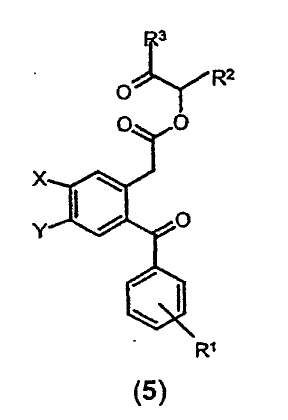 in which
X, Y, R¹, R² and R³ have the abovementioned meaning,
b) by condensation with ammonia or an ammonia donor to form the oxazole of general formula **6**, 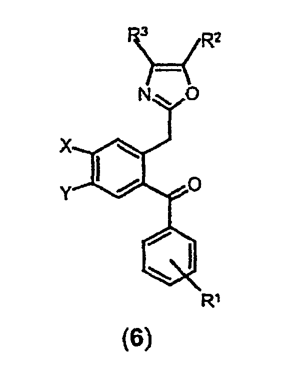 in which
X, Y, R¹, R² and R³ have the abovementioned meaning, and subsequent hydrazinolysis to form the compounds of general formula 1 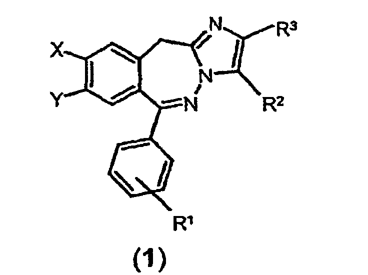 in which
X, Y, R¹, R² and R³ have the abovementioned meaning.

2. Process according to claim 1, wherein compounds of general formula **5** are reacted using a single-pot reaction in the presence of ammonium acetate and ammonia or an ammonia donor at 0-150°C in a solvent or solvent mixtures and then with hydrazine at 0-200°C, optionally in an autoclave, to give the compounds of general formula 1.

3. Process according to claim 1 or 2 for the production of 2,3-dimethyl-6-phenyl-(12*H*)-[1,3]dioxolo[4,5-h]imidazo[1,2-c][2,3]benzodiazepine
2,3-dimethyl-6-(4-chlorophenyl)-(12*H*)-[1,3]dioxolo[4,5-h]imidazo[1,2-c][2,3]benzodiazepine
2,3-dimethyl-6-(4-nitrophenyl)-(12*H*)-[1,3]dioxolo[4,5-h]imidazo[1,2-c][2,3]benzodiazepine
2,3-dimethyl-6-(4-methoxyphenyl)-(12*H*)-[1,3]dioxolo[4,5-h]imidazo[1,2-c][2,3]benzodiazepine
2,3-diethyl-6-phenyl-(12*H*)-[1,3]dioxolo[4,5-h]imidazo[1,2-c][2,3]benzodiazepine
2,3-diphenyl-6-phenyl-(12*H*)-[1,3]dioxolo[4,5-h]imidazo[1,2-c][2,3]benzodiazepine.

4. Phenylacetic acid ester of general formula **5**, 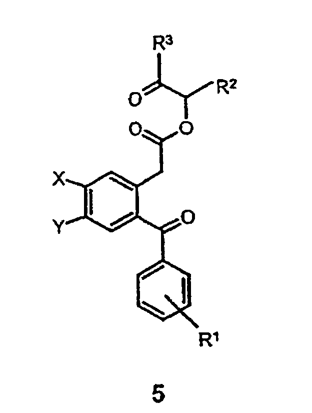 in which
R¹ = hydrogen, C₁-C₆-alkyl, nitro, halogen, cyano, C₁-C₄-alkoxy, -CF₃, hydroxy or C₁-C₆-alkanoyloxy,
R² and R³ are the same or different and mean hydrogen, halogen, C₁-C₆-alkoxy, hydroxy, cyano, C₁-C₆-alkanoyl, C₂-C₆-alkynyl, C₂-C₆-alkenyl; C₁-C₆-alkyl or C₃-C₇-cycloalkyl optionally substituted by halogen, hydroxy or C₁₋₆-alkoxy; or an aryl or hetaryl radical that is optionally substituted by halogen, C₁₋₄-alkoxy or C₁₋₄-alkyl,
X = hydrogen or halogen,
Y = C₁-C₆-alkoxy or
X and Y together mean -O-(CH₂)ₙ-O- with
n = 1, 2 or 3.

5. Oxazole derivatives of general formula **6,** 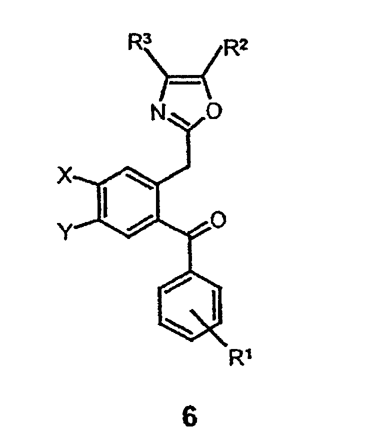 in which
R¹ = hydrogen, C₁-C₆-alkyl, nitro, halogen, cyano, C₁-C₄-alkoxy, -CF₃, hydroxy or C₁-C₆-alkanoyloxy,
R² and R³ are the same or different and mean hydrogen, halogen, C₁-C₆-alkoxy, hydroxy, cyano, C₁-C₆-alkanoyl, C₂-C₆-alkynyl, C₂-C₆-alkenyl; C₁-C₆-alkyl or C₃-C₇-cycloalkyl optionally substituted by halogen, hydroxy or C₁₋₆-alkoxy; or an aryl or hetaryl radical optionally substituted by halogen, C₁₋₄-alkoxy or C₁₋₄-alkyl,
X = hydrogen or halogen,
Y = C₁-C₆-alkoxy or
X and Y together mean -O-(CH₂)ₙ-O- with
n = 1, 2 or 3.

## Revendications

1. Procédé en vue de la préparation d'imidazo[1,2-c]-[2,3]benzodiazépines de la formule générale 1 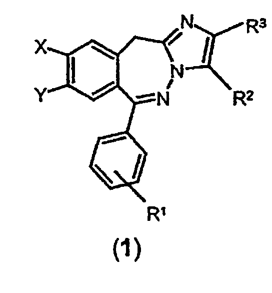 où
R¹ = hydrogène, C₁-C₆-alkyle, nitro, halogène, cyano, C₁-C₄-alkoxy, -CF₃, hydroxy ou C₁-C₆-alcanoyloxy,
R² et R³ sont identiques ou différents et représentent l'hydrogène, un halogène, les radicaux C₁-C₆-alkoxy, hydroxy, cyano, C₁-C₆-alcanoyle, C₂-C₆-alcynyle, C₂-C₆-alcényle, C₁-C₆-alkyle, substitué, le cas échéant, par un halogène, un radical hydroxy ou un radical C₁₋₆-alkoxy, C₃-C₇-cycloalkyle ou un radical aryle ou un radical hétéroaryle substitué, le cas échéant, par un halogène, un radical C₁₋₄-alkoxy ou C₁₋₄-alkyle,
X = l'hydrogène ou un halogène,
Y = C₁-C₆-alkoxy ou
X et Y, conjointement, désignent -O-(CH₂)ₙ-O- avec
n = 1, 2 ou 3,
à partir d'acides phénylacétiques de la formule générale 4, 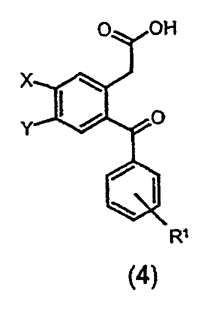 où
X, Y et R¹ ont la signification suscitée,
a) par estérification avec un alcool de la formule 5a 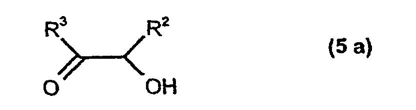 pour former un ester de l'acide phénylacétique de la formule générale 5, 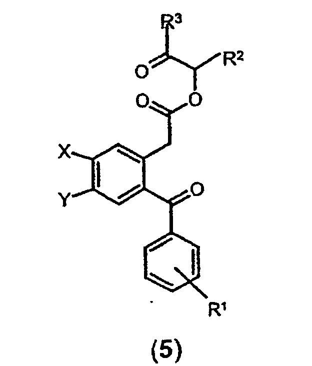 où
X, Y, R¹, R² et R³ ont la signification suscitée,
b) par condensation à l'aide d'ammoniac ou d'un donneur d'ammoniac, pour former l'oxazole de la formule générale 6, 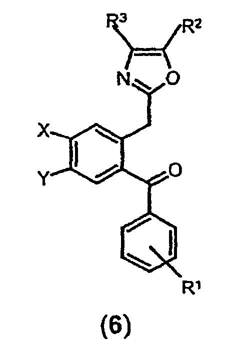 où
X, Y, R¹, R² et R³ ont la signification suscitée et par hydrazinolyse subséquente, pour former les composés de la formule générale 1 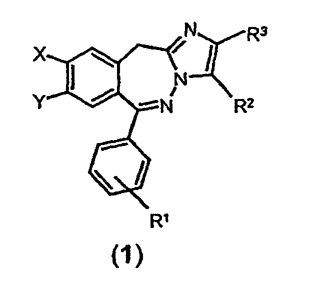 où
X, Y, R¹, R² et R³ ont la signification suscitée.

2. Procédé selon la revendication 1, les composés de la formule générale 5 étant convertis dans les composés de la formule générale 1 par l'intermédiaire d'une réaction dans une enceinte, par réaction en présence d'acétate d'ammonium et d'ammoniac ou d'un donneur d'ammoniac, à une température de 0 - 150°C dans le solvant ou dans des mélanges de solvants et ensuite à l'aide d'hydrazine, à une température de 0 - 200°C, le cas échéant, dans un autoclave.

3. Procédé selon la revendication 1 ou 2, en vue de la préparation
de 2,3-diméthyl-6-phényl-(12*H*)-[1,3]dioxolo[4,5-h]imidazo[1,2-c][2,3]benzodiazépine,
de 2,3-diméthyl-6-(4-chloro-phényl)-(12*H*)-[1,3]dioxolo[4,5-h]imidazo[1,2-c][2,3]benzodiazépine,
de 2,3-diméthyl-6-(4-nitro-phényl)-(12*H*)-[1,3]dioxolo[4,5-h]imidazo[1,2-c][2,3]benzodiazépine,
de 2,3-diméthyl-6-(4-méthoxy-phényl)-(12*H*)-[1,3]dioxolo[4,5-h]imidazo[1,2-c][2,3]benzodiazépine,
de 2,3-diéthyl-6-phényl-(12*H*)-[1,3]dioxolo[4,5-h]imidazo[1,2-c][2,3]benzodiazépine,
de 2,3-diphényl-6-phényl-(12*H*)-[1,3]dioxolo[4,5-h]imidazo[1,2-c][2,3]benzodiazépine.

4. Ester de l'acide phénylacétique de la formule générale 5, 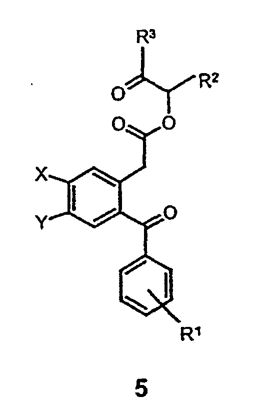 où
R¹ = hydrogène, C₁-C₆-alkyle, nitro, halogène, cyano, C₁-C₄-alkoxy, -CF₃, hydroxy ou C₁-C₆-alcanoyloxy,
R² et R³ sont identiques ou différents et représentent l'hydrogène, un halogène, les radicaux C₁-C₆-alkoxy, hydroxy, cyano, C₁-C₆-alcanoyle, C₂-C₆-alcynyle, C₂-C₆-alcényle, C₁-C₆-alkyle, substitué, le cas échéant, par un halogène, un radical hydroxy ou un radical C₁₋₆-alkoxy, C₃-C₇-cycloalkyle ou un radical aryle ou un radical hétéroaryle substitué, le cas échéant, par un halogène, un radical C₁₋₄-alkoxy ou C₁₋₄-alkyle,
X = l'hydrogène ou un halogène,
Y = C₁-C₆-alkoxy ou
X et Y, conjointement, désignent -O-(CH₂)ₙ-O- avec
n = 1, 2 ou 3.

5. Dérivés d'oxazole de la formule générale 6, 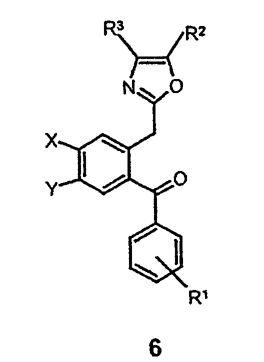 où
R¹ = hydrogène, C₁-C₆-alkyle, nitro, halogène, cyano, C₁-C₄-alkoxy, -CF₃, hydroxy ou C₁-C₆-alcanoyloxy,
R² et R³ sont identiques ou différents et représentent l'hydrogène, un halogène, les radicaux C₁-C₆-alkoxy, hydroxy, cyano, C₁-C₆-alcanoyle, C₂-C₆-alcynyle, C₂-C₆-alcényle, C₁-C₆-alkyle, substitué, le cas échéant, par un halogène, un radical hydroxy ou un radical C₁₋₆-alkoxy, C₃-C₇-cycloalkyle ou un radical aryle ou un radical hétéroaryle substitué, le cas échéant, par un halogène, un radical C₁₋₄-alkoxy ou C₁₋₄-alkyle,
X = l'hydrogène ou un halogène,
Y = C₁-C₆-alkoxy ou
X et Y, conjointement, désignent -O-(CH₂)ₙ-O- avec
n = 1, 2 ou 3.
